# DEMANDE DE BREVET EUROPEEN

(11) **EP 4 663 174 A1**
(43) Date de publication de la demande: **17.12.2025**
(21) Numéro de dépôt: 25181281.4
(22) Date de dépôt: 06.06.2025
(51) Int. Cl.: A61F 9/02, G02C 1/00, G02C 9/00

(54) **MASQUE DE PROTECTION OCULAIRE**

(30) Priorité: 10.06.2024 FR 2406064
(71) Demandeur: Skis Rossignol - Club Rossignol, 38430 Saint-Jean-de-Moirans (FR)
(72) Inventeur: MORVAN, Thomas, 38450 Le Gua (FR)
(74) Mandataire: Novaimo

(57) **Abrégé**

Masque (1) de protection oculaire comprenant une monture (7) et un système d'écran (14) fixé à la monture, le système d'écran comprenant un premier écran (2) et un support (13) fixé au premier écran, le premier écran recouvrant frontalement la monture et le support, caractérisé en ce que le support comprend :
- une surface avant (15) fixée contre une face arrière (16) du premier écran,
- une surface de retenue (23) s'étendant sensiblement parallèlement au premier écran à l'arrière du premier écran,
la surface de retenue et un bord externe (3) du premier écran formant ensemble une gorge radiale (22) coopérant avec la monture (7) pour retenir le système d'écran à la monture.

## Description

### Domaine Technique de l'invention

L'invention concerne un masque de protection oculaire destiné à protéger les yeux, tel qu'un masque de ski. Le masque de protection oculaire est notamment destiné à protéger les yeux contre les rayons du soleil et/ou contre les projections lors de la pratique d'une activité sportive. L'invention porte notamment sur un masque de protection oculaire de type « sans monture apparente », c'est-à-dire un masque de protection oculaire comprenant un écran recouvrant frontalement une monture agencée derrière l'écran.

### Etat de la technique antérieure

Pour protéger les yeux contre le soleil et/ou contre des projections lors de la pratique d'une activité, il est courant de porter un masque de protection oculaire équipé d'un écran de protection transparent ou translucide. Le port d'un tel masque est particulièrement recommandé pour la pratique d'un sport de glisse sur neige comme le ski ou le snowboard, mais également pour la pratique du vélo tout terrain, de la moto ou encore des activités lors desquelles des projectiles à haute vitesse sont utilisés, comme le tir. Un tel masque est plaqué contre le visage autour des yeux et offre ainsi une protection oculaire particulièrement efficace.

On connait en particulier des masques de protection oculaire sans monture apparente, couramment dénommés par l'anglicisme « frameless ». De tels masques comprennent un écran de protection recouvrant frontalement une monture agencée derrière l'écran. Ainsi, en vue de face, c'est-à-dire lorsqu'on regarde le porteur d'un tel masque en face à face, la monture est complètement cachée derrière l'écran, et seul l'écran est apparent. Un tel masque offre un champ de vision agrandi et présente un aspect esthétique original. La publication WO2012178049A1 divulgue un tel masque.

La fixation d'un tel écran de protection à la monture est complexe à réaliser. En particulier, les moyens de fixation connus de l'état de la technique pour fixer l'écran de protection à la monture sont relativement volumineux. Par conséquent, l'écran de protection se trouve assez éloigné du visage pour disposer d'assez de place pour intégrer ces moyens de fixation. Cela pénalise l'aspect esthétique de tels masques et leur champ de vision. De plus, les moyens de fixation connus de l'état de la technique sont relativement fragiles et peuvent facilement être endommagés, par exemple lors de la manipulation d'un tel masque ou en cas de chute de leur utilisateur. L'écran pourrait alors se détacher de la monture de façon accidentelle ou involontaire, exposant le visage de l'utilisateur à l'environnement extérieur, pouvant aller jusqu'à le blesser.

### Présentation de l'invention

Le but de l'invention est de fournir un masque de protection oculaire remédiant aux inconvénients ci-dessus et améliorant les masques de protection oculaire connus de l'art antérieur.

Plus précisément, un premier objet de l'invention est un masque de protection oculaire sans monture apparente particulièrement robuste, compact, et simple à fabriquer.

### Résumé de l'invention

L'invention se rapporte à un masque de protection oculaire comprenant une monture et un système d'écran fixé à la monture, le système d'écran comprenant un premier écran et un support fixé au premier écran, le premier écran recouvrant frontalement la monture et le support, le support comprenant :
- une surface avant fixée contre une face arrière du premier écran,
- une surface de retenue s'étendant sensiblement parallèlement au premier écran à l'arrière du premier écran,
la surface de retenue et un bord externe du premier écran formant ensemble une gorge radiale coopérant avec la monture pour retenir le système d'écran à la monture.

Le support peut comprendre en outre une surface arrière s'étendant en vis-à-vis de la monture, la surface de retenue s'étendant entre la surface avant du support et la surface arrière du support.

La monture peut comprendre une rainure radiale, la surface de retenue étant insérée dans la rainure radiale.

La surface de retenue peut s'étendre le long d'au moins 50%, de préférence au moins 80%, d'un bord externe de l'écran.

La surface de retenue peut comprendre au moins une encoche, et la monture peut comprendre au moins une protubérance de forme complémentaire à la forme de l'au moins une encoche, l'au moins une protubérance étant emboîtée dans l'au moins une encoche.

L'au moins une encoche peut comprendre une entrée rétrécie.

L'au moins une encoche peut comprendre :
- au moins une première encoche formée sur une portion de la surface de retenue destinée à s'étendre autour du nez,
- au moins une deuxième encoche formée sur une portion de la surface de retenue destinée à s'étendre en vis-à-vis d'une pommette, et
- au moins une troisième encoche formée sur une portion de la surface de retenue destinée à s'étendre en vis-à-vis du front.

La surface de retenue peut s'étendre au moins :
- dans une partie inférieure du support destinée à s'étendre en vis-à-vis des pommettes et autour du nez,
- dans une partie supérieure du support destinée à s'étendre en vis-à-vis du front,
- dans une partie latérale gauche du support destinée à s'étendre en vis-à-vis d'un côté extérieur de l'œil gauche, et
- dans une partie latérale droite du support destinée à s'étendre en vis-à-vis d'un côté extérieur de l'œil droit.

La monture peut être davantage déformable que le système d'écran, seule la monture étant destinée à se déformer pour assembler ou désassembler le système d'écran à la monture.

Le masque peut comprendre une bande élastique configurée pour maintenir le masque de protection oculaire sur la tête d'une personne, la bande élastique étant fixée à la monture.

L'ensemble du bord externe du premier écran peut être visible depuis l'extérieur du masque.

Le système d'écran peut comprendre un deuxième écran s'étendant parallèlement au premier écran à l'arrière du premier écran, le deuxième écran étant maintenu par le support.

### Présentation des figures

Ces objets, caractéristiques et avantages de la présente invention seront exposés en détail dans la description suivante d'un mode de réalisation particulier fait à titre non-limitatif en relation avec les figures jointes parmi lesquelles :
La figure 1 est une vue de face avant d'un masque de protection oculaire selon un mode de réalisation de l'invention.
La figure 2 est une vue de dessus du masque de protection oculaire.
La figure 3 est une vue de côté du masque de protection oculaire.
La figure 4 est une vue en perspective d'un support d'écran du masque de protection oculaire.
La figure 5 est une vue de face arrière d'un écran du masque de protection oculaire.
La figure 6 est une vue de face arrière d'un système d'écran du masque de protection oculaire, le système d'écran comprenant le support de la figure 4 et l'écran de la figure 5.
La figure 7 est une vue en coupe d'une partie du système d'écran du masque de protection oculaire.
La figure 8 est une vue en coupe d'une partie du système d'écran et d'une monture du masque de protection oculaire, la monture coopérant avec le système d'écran pour maintenir le système d'écran.
La figure 9 est une vue en perspective d'un corps de la monture du masque de protection oculaire.
La figure 10 est une vue de détail d'une encoche formée dans une aile du support d'écran du masque de protection oculaire.
La figure 11 est une vue en éclaté du masque de protection oculaire, le masque comprenant un premier écran et un deuxième écran.

### Description détaillée

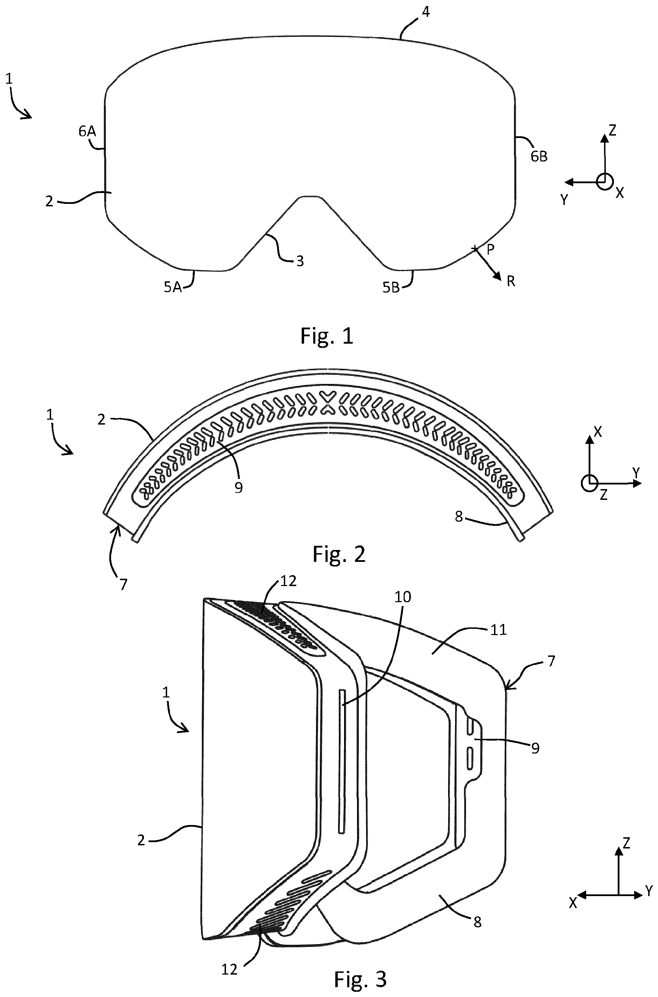

La figure 1 illustre un masque de protection oculaire 1 selon un mode de réalisation de l'invention. Le masque de protection oculaire 1 est destiné à protéger les yeux du soleil et/ou de projections lors de la pratique d'une activité sportive telle qu'un sport de glisse sur neige comme le ski alpin ou le snowboard, le vélo tout terrain, la moto, ou encore une activité lors de laquelle des projectiles à haute vitesse sont utilisés, comme le tir. Lorsqu'il est utilisé pour la pratique d'un sport de glisse sur neige, un tel masque peut être dénommé « masque de ski ».

Le masque de protection oculaire 1, plus simplement dénommé « masque 1 » par la suite, comprend un écran 2 de protection transparent ou translucide au travers duquel un utilisateur est apte à observer l'environnement. L'écran 2 s'étend donc dans un champ de vision d'un porteur du masque, et notamment vis-à-vis des deux yeux de son utilisateur. L'écran 2 peut notamment être teinté pour assombrir une perception de l'environnement et ainsi protéger un porteur du masque des rayons du soleil. Alternativement, l'écran 2 peut être transparent pour simplement protéger les yeux des projections sans assombrir une perception de l'environnement. L'écran 2, que l'on peut également dénommer « verre », se trouve préférentiellement en matière plastique, notamment en polycarbonate

Le masque 1 est de type « sans monture apparente ». Seul l'écran 2 est directement apparent lorsqu'on observe le masque 1 en vue de face, c'est à dire selon le point de vue de la figure 1. Le masque 1 comprend ainsi de préférence une face avant lisse, non recouverte localement par une partie de la monture ou tout autre élément structurel destiné à maintenir l'écran. Le masque 1 ne comprend donc pas de replis ou recoins sur sa face avant, à l'interface entre l'écran 2 et un tel élément structurel. On évite ainsi que des particules puissent s'accumuler dans de tels recoins. L'écran 2 est ainsi particulièrement simple à nettoyer et offre un aspect esthétique original.

En remarque, dans l'hypothèse où l'écran 2 est suffisamment transparent, il demeure néanmoins possible de distinguer des composants du masque 1 autres que son écran 2 en regardant au travers de l'écran. Un masque dont une partie de la monture serait visible, en vue de face, seulement au travers de l'écran 2 est néanmoins considéré comme un masque « sans monture apparente » au sens de l'invention.

Le masque 1 est destiné à être plaqué sur le visage autour des yeux. Selon la vue de face telle que représentée sur la figure 1, l'écran 2 comprend une forme grossièrement rectangulaire avec une échancrure pour contourner le nez. L'écran 2 comprend un bord externe 3, ou contour 3, formé notamment par :
- un bord supérieur 4 s'étendant en vis-à-vis du front,
- deux bords inférieurs 5A, 5B de part et d'autre de l'échancrure contournant le nez, et s'étendant en vis-à-vis des pommettes, et
- des bords latéraux 6A, 6B s'étendant sensiblement verticalement sur les côtés des yeux.

De préférence, au moins 80% du bord externe 3 de l'écran, voire au moins 90% du bord externe 3 de l'écran pourrait être visible. Selon le mode de réalisation présenté, le bord externe 3 de l'écran 2 est entièrement visible depuis l'extérieur du masque 1, c'est-à-dire que 100% du bord externe 3 de l'écran est visible et non recouvert par un élément structurel du masque.

Selon une vue de dessus, illustrée sur la figure 2, on observe que le masque 1 présente une forme arrondie, destinée à épouser la forme de la tête et du visage du porteur du masque. D'une manière générale, l'écran 2 peut, par exemple, présenter la forme d'une portion d'un cylindre de révolution, voire la forme d'une portion d'une sphère.

On définit l'axe longitudinal X comme l'axe du regard d'un porteur du masque lorsqu'il regarde droit devant lui. L'axe longitudinal est un axe horizontal, orienté de l'arrière vers l'avant. On définit l'axe vertical Z comme l'axe dans lequel s'étend un porteur du masque lorsqu'il se tient debout. L'axe vertical Z est perpendiculaire à l'axe longitudinal X. L'axe vertical Z est orientée de bas en haut. Enfin, on définit l'axe transversal Y comme l'axe perpendiculaire aux axes X et Z, et orienté de gauche à droite selon le point de vue d'un porteur du masque 1. Les axes X, Y et Z forment un repère orthogonal. Pour un point P donné du bord externe 3 de l'écran 2, on définit l'axe radial R comme l'axe à la fois parallèle à l'écran 2 au niveau du point P considéré et perpendiculaire au bord externe 3 au niveau du point P. L'axe radial R est orienté dans une direction centrifuge.

Comme cela est visible sur les figures 2 et 3, le masque 1 comprend une monture 7 supportant l'écran 2. La monture 7 est équipée d'une bande élastique (non représentée) destinée à passer derrière la tête ou derrière un casque pour plaquer le masque 1 contre le visage de son porteur. La bande élastique comprend une première extrémité fixée à un bord gauche de la monture 7 et une deuxième extrémité fixée à un bord droit de la monture. Plus précisément, la monture 7 comprend un corps 8 et deux moyens de fixation 9 gauche et droite coopérant avec les deux extrémités de la bande élastique. Les deux moyens de fixation 9 sont intégrés au corps 8. La bande élastique passe au travers du corps 8 par deux fentes 10 gauche et droite prévues à cet effet. La monture 7 comprend avantageusement une surface d'appui 11 circonférentielle, en matériau souple, apte à se déformer au contact du visage. Comme expliqué précédemment, la monture 7 est recouverte frontalement par l'écran 2. Par « frontalement » on comprend que la monture 7 est recouverte par l'écran en vue de face.

Avantageusement, la monture 7 comprend aussi des moyens d'aération 12, par exemple sous la forme d'une ou plusieurs grilles, fixées au corps 8 ou formées directement dans le corps 8. Les moyens d'aération 12 peuvent, par exemple être agencés au niveau d'une partie supérieure et/ou d'une partie inférieure de la monture 7, entre l'écran 2 et la surface d'appui 11. Les moyens d'aération 12 permettent d'éviter la formation de buée sur l'écran 2.

Le corps 8 de la monture 7 est avantageusement constitué d'un matériau souple et élastique. La surface d'appui 11 peut ainsi être formée directement sur le corps 8. Le corps 8 peut ainsi être destiné à prendre appui directement contre le visage. Alternativement, la surface d'appui 11 peut être formée dans un élément distinct du corps 8 et rapporté, par exemple collé, sur le corps 8. Un tel élément rapporté peut, par exemple, être un élément en mousse ou en tissu.

Selon un mode de réalisation, le corps 8 de la monture peut être constitué de polyuréthane thermoplastique (TPU). Le matériau constituant le corps peut comprendre une dureté comprise entre 80 et 90 Shore A mesurée selon la norme ASTM D2240.

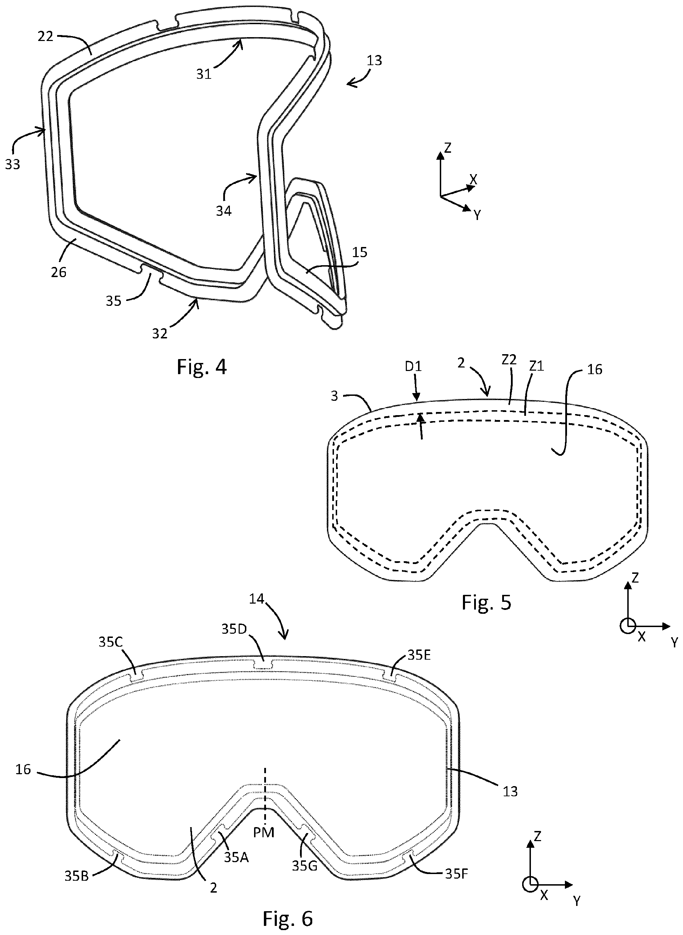

On décrit à présent un moyen de fixation de l'écran 2 à la monture 7 selon un mode de réalisation de l'invention, à l'appui des figures 4 à 10. L'écran 2 est fixé à la monture 7 au moyen d'un support d'écran 13, plus simplement dénommé « support 13 » par la suite. Le support 13 est illustré sur la figure 4. L'écran 2 est illustré sur la figure 5. On appelle « système d'écran 14 », l'ensemble formé par l'écran 2 et le support 13. Le système d'écran 14 est illustré sur la figure 6. En particulier, l'écran 2 est fixé sur une face externe du support 13. L'écran 2 peut ainsi être qualifié d'écran externe.

L'écran 2 recouvre frontalement non seulement la monture 7 comme expliqué précédemment, mais également le support 13. Ainsi, le support 13 ne dépasse pas radialement du bord externe 3 de l'écran. Le support 13 est donc invisible en vue de face depuis l'extérieur du masque 1 ou seulement visible au travers de l'écran 2. Comme nous le verrons par la suite, le support 13 est également invisible en observant le masque 1 sur ses côtés puisqu'il est recouvert par la monture 7. Le support 13 comprend une forme générale qui épouse globalement la forme du bord externe 3 de l'écran 2. Le support 13 comprend notamment une ligne fermée délimitant une ouverture centrale refermée par l'écran 2. Selon le mode de réalisation présenté, la ligne que suit le support 13 est ininterrompue et suit donc la totalité du bord externe 3 de l'écran. Selon une variante de réalisation, le support pourrait s'étendre seulement le long d'une partie du bord externe 3 de l'écran 2, de préférence sur au moins 50% de la longueur du bord externe 3, voire au moins 80% de la longueur du bord externe 3. Selon une autre variante de réalisation, le support 13 pourrait être formé de plusieurs éléments de support, séparés les uns des autres le long du bord externe 3 de l'écran 2.

Le support 13 est de préférence un élément monobloc, de préférence en plastique, par exemple un élément en plastique obtenu par moulage. Le support 3 peut être constitué d'un polymère thermoplastique tel que de l'acrylonitrile butadiène styrène (ABS), du polycarbonate (PC), ou encore un mélange de ces deux matériaux.

Le support 13 est fixé à l'écran 2. En particulier, le support 13 comprend une surface avant 15 fixée contre une face arrière 16 de l'écran 2. De préférence, la surface avant 15 du support 13 est collée contre la face arrière 16 de l'écran 2. Selon une variante de réalisation, le support 13 et l'écran 2 pourraient être fixés ensemble différemment, par exemple par emboîtement ou par soudure. Alternativement, le support 13 et l'écran 2 pourraient même former une seule et même pièce monolithique.

Une première zone Z1 de la face arrière 16 de l'écran 2 contre laquelle la surface avant 15 du support 13 est fixée est identifiée par des lignes en pointillés sur la figure 5. Cette première zone Z1 est distante du bord externe 3 de l'écran 2 d'une distance D1 non nulle par exemple, au moins 5mm. Entre la première zone Z1 et le bord externe 3 de l'écran 2, la face arrière 16 comprend une deuxième zone Z2. La deuxième zone comprend donc une largeur non nulle. L'écran est en appui direct contre la monture 7, notamment contre le corps 8 de la monture 7, par l'intermédiaire de sa deuxième zone Z2. La deuxième zone Z2 de l'écran 2 permet ainsi de masquer la monture 7 en vue de face du masque 1. Autrement dit, la deuxième zone Z2 de l'écran recouvre au moins en partie une face externe de la monture.

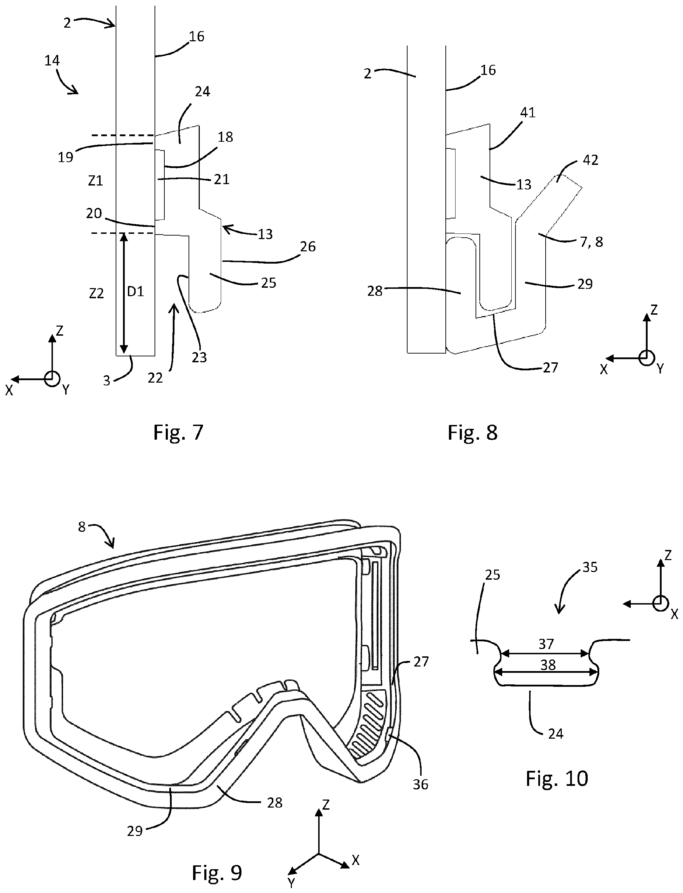

La figure 7 illustre par une vue en coupe selon un plan médian PM une partie du système d'écran 14. Le plan médian PM est identifié sur la figure 6. Il s'agit d'un plan parallèle aux axes X et Z, passant par un centre du masque 1. On observe que la surface avant 15 du support 13 comprend une zone en retrait 18 encadrée par un rebord interne 19 et un rebord externe 20. Les rebords 19 et 20 sont en appui directement contre la face arrière 16 de l'écran 2. La zone en retrait 18 permet de former un volume libre 21 entre la face arrière 16 de l'écran 2 et le support 13. Ce volume libre 21 est destiné à recevoir de la colle pour la fixation de l'écran 2 au support 13. Ceci permet de circonscrire la surface de l'écran recevant de la colle et donc permet d'éviter que de la colle ne fuite sur une zone utile de l'écran. Avantageusement, les rebords 19 et 20 sont en appui contre l'écran sur l'ensemble du pourtour de l'écran, sans interruption. Le cordon de colle permettant de fixer le support 13 à l'écran 2 suit ainsi une ligne fermée et continue. On améliore ainsi la tenue du support 13 sur l'écran 2. Alternativement, un ruban autocollant, par exemple un joint autocollant, pourrait être disposé dans la zone en retrait 18, entre l'écran 2 et le support 13. Selon une variante de réalisation, la surface avant 15 pourrait comprendre des discontinuités. En remarque, l'écran 2 peut avantageusement être teinté au moins au niveau des zones Z1 et Z2 afin de masquer le support 13 et la monture 7.

Le support 13 comprend aussi une surface de retenue 23, s'étendant parallèlement à la surface avant 15, et décalée vers l'arrière par rapport à la surface avant 15. La surface de retenue 23 s'étend également parallèlement à l'écran 2. La surface de retenue 23 coopère avec la monture 7, notamment avec le corps 8 de la monture 7, pour retenir le système d'écran 14 à la monture. En particulier, un effort de réaction de la surface de retenue 23 contre le corps 8 de la monture 7 empêche la dissociation par l'avant du système d'écran 14 de la monture 7.

La surface de retenue 23 s'étend plus à l'arrière que la surface avant 15. Ainsi, une gorge radiale 22 est formée entre la surface de retenue 23 du support 13 et le bord externe 3 de l'écran 2. Comme nous allons le voir par la suite, le corps 8 comprend une paroi avant 28 insérée à l'intérieur de cette gorge radiale 22. En remarque, la branche de la gorge radiale 22 formée par l'écran 2 est plus haute que la branche de la gorge radiale 22 formée par le support 13. Ceci permet à écran 2 de bien recouvrir non seulement le support 13 mais également la monture 7.

Selon une autre approche, le support 13 comprend une embase 24 fixée à l'écran, et une aile 25 s'étendant depuis l'embase 24 parallèlement à l'écran 2 en direction du bord externe 3 de l'écran. L'aile 25 s'étend donc radialement dans une direction centrifuge. L'embase 24 comprend la surface avant 15 du support, et l'aile 25 comprend la surface de retenue 23.

La surface de retenue 23 s'étend le long du bord externe 3 de l'écran, de préférence le long d'au moins 50% de la longueur du bord externe 3 de l'écran, voire au moins 80% de la longueur du bord externe 3 de l'écran. Plus la surface de retenue 23 s'étend le long d'une proportion importante du bord externe 3 de l'écran, meilleure est la retenue conférée par la coopération de cette surface de retenue avec la monture 7. Selon un mode de réalisation, la surface de retenue 23 s'étend à 360° autour de l'écran 2. Une surface de retenue s'étendant le long d'une proportion importante du bord externe 3 de l'écran permet donc d'envisager une surface de retenue de faible hauteur suivant l'axe radial R et/ou l'utilisation de matériau plus souple et donc plus confortable pour réaliser le corps 8 de la monture 7.

De préférence, la surface de retenue 23 s'étend au moins :
- dans une partie inférieure 31 du support destinée à s'étendre en vis-à-vis des pommettes et autour du nez d'un porteur du masque 1,
- dans une partie supérieure 32 du support destinée à s'étendre en vis-à-vis du front du porteur du masque 1,
- dans une partie latérale gauche 33 du support destinée à s'étendre en vis-à-vis d'un côté extérieur de l'œil gauche du front du porteur du masque 1, et
- dans une partie latérale droite 34 du support destinée à s'étendre en vis-à-vis d'un côté extérieur de l'œil droit du front du porteur du masque 1. Le système d'écran 14 est ainsi retenu à la monture 7 sur ses différents côtés, ce qui permet d'obtenir un maintien homogène et robuste du système d'écran 14.

Le support 13 comprend également une surface arrière 26 s'étendant en vis-à-vis de la monture 7, notamment en vis-à-vis du corps 8 de la monture 7. La surface arrière 26 s'étend parallèlement à l'écran 2. La surface arrière 26 peut se trouver, au moins localement, en appui contre la monture 7. La surface arrière 26 peut également s'étendre, au moins localement, à une distance non nulle de la monture comme cela apparait sur la figure 8. La distance D2 séparant la surface avant 15 de la surface arrière 26 du support peut être, de préférence, inférieure ou égale à 5mm, voire inférieure ou égale à 3mm. Le support 13 est donc particulièrement compact selon l'axe longitudinal X. La surface arrière 26 et la surface de retenue 23 du support sont deux faces opposées de l'aile 25. La surface arrière 26 est tournée vers l'arrière du masque 1 et la surface de retenue 23 est tournée vers l'avant. La surface arrière 26 est globalement plane. En cas d'un effort axial exercé sur l'écran 2, une large surface de contact est ainsi obtenue entre la monture 7 et le support 13, et le support 13 ne risque pas d'être endommagé.

Comme cela est visible sur les figures 8 et 9, le corps 8 de la monture 7 comprend une rainure radiale 27. La rainure radiale 27 s'étend parallèlement à l'axe radial R sur l'ensemble du pourtour de la monture 7. Cette rainure radiale 27 est formée par une paroi avant 28 et une paroi arrière 29 s'étendant parallèlement à la paroi avant 28. L'aile 25 du support 13 est insérée dans la rainure radiale 27 de la monture. Ainsi le support 13 est masqué par la monture 7 sur les côtés du masque 1. La surface de retenue 23 du support étant formée sur l'aile 25, la surface de retenue 23 est donc également insérée dans la rainure radiale 27 de la monture.

Une face avant de la paroi avant 28 de la monture est en appui ou susceptible d'être en appui contre la deuxième zone Z2 de la face arrière 16 de l'écran 2. Une face arrière de la paroi avant 28 est en appui ou susceptible d'être en appui contre la surface de retenue 23 du support. Avantageusement, l'épaisseur de la paroi avant 28 est sensiblement égale à la distance séparant la surface de retenue 23 du support de la face arrière 16 de l'écran. Une face avant de la paroi arrière 29 est en appui ou susceptible d'être en appui contre la surface arrière 26 du support. La rainure radiale 27 présente avantageusement une largeur constante sur le pourtour de la monture 7.

En référence aux figures 4, 6 et 9, on observe que la surface de retenue 23 du support comprend un ensemble d'encoches 35. Le corps 8 de la monture 7 comprend un ensemble de protubérances 36 de forme complémentaire à la forme des encoches 35. Chaque protubérance 36 de la monture est ainsi emboîtée dans une encoche 35 du support. Ceci permet d'améliorer le maintien du système d'écran 14 à la monture 7. Les encoches 35 sont des interruptions locales dans la surface de retenue 23, et même les uniques interruptions de la surface de retenue 23. Les protubérances 36 sont agencées au fond de la rainure radiale 27, notamment entre les parois avant 28 et arrière 29 de la monture.

Toutes les encoches 35 peuvent avoir des formes identiques et toutes les protubérances 36 peuvent avoir des formes identiques. En particulier, comme cela est illustré sur la figure 10, chaque encoche 35 comprend avantageusement une entrée 37 rétrécie, c'est-à-dire une entrée 37 plus étroite que le fond 38 de l'encoche 35. L'engagement de la protubérance 36 à l'intérieur de l'encoche 35 requiert ainsi une déformation élastique de la protubérance 36 et/ou de l'encoche 35. Un tel artifice améliore encore le maintien du système d'écran 14 sur la monture 7 sans pénaliser l'encombrement global du masque 1.

Selon le mode de réalisation présenté, la surface de retenue 23 comprend sept encoches 35 distantes les unes des autres. Quatre encoches sont formées dans la partie inférieure 31 du support et trois encoches sont formées dans la partie supérieure 32 du support.

Plus précisément, le support 13 comprend :
- une première encoche 35A formée sur une portion de la surface de retenue destinée à s'étendre autour du nez, notamment à droite du nez du porteur du masque,
- une deuxième encoche 35B formée sur une portion de la surface de retenue destinée à s'étendre en vis-à-vis d'une pommette droite du porteur du masque,
- une troisième encoche 35C formée sur une portion de la surface de retenue destinée à s'étendre en vis-à-vis du front, notamment sensiblement au-dessus de l'œil droit du porteur du masque,
- une quatrième encoche 35D formée sur une portion de la surface de retenue destinée à s'étendre en vis-à-vis du front, notamment au-dessus du nez,
- une cinquième encoche 35E formée sur une portion de la surface de retenue destinée à s'étendre en vis-à-vis du front, notamment sensiblement au-dessus de l'œil gauche du porteur du masque,
- une sixième encoche 35F formée sur une portion de la surface de retenue destinée à s'étendre en vis-à-vis d'une pommette gauche du porteur du masque, et
- une septième encoche 35G formée sur une portion de la surface de retenue destinée à s'étendre autour du nez, notamment à gauche du nez du porteur du masque.

Le corps 8 comprend sept protubérances 36 positionnées en vis-à-vis des sept encoches.

En variante, le nombre d'encoches 35 et le nombre de protubérances 36 correspondantes pourraient être différents. De préférence, le masque comprend entre cinq et dix encoches et autant de protubérances correspondantes, de manière à obtenir un bon maintien du système d'écran 14 à la monture 7, tout en conservant un assemblage simple entre ces deux éléments.

Le système d'écran 14 est avantageusement fixé à la monture 7 de manière amovible, c'est-à-dire de manière réversible. En particulier le système d'écran 14 peut être assemblé et désassemblé de la monture 7 par l'utilisateur du masque 1, sans utiliser d'outil. Avantageusement, le masque 1 peut être livré avec plusieurs systèmes d'écran14 de forme identique. De tels systèmes d'écran peuvent se distinguer les uns des autres par l'opacité de leur écran (par exemple pour s'adapter à des conditions climatiques ou de visibilité différentes) et/ou par leur aspect esthétique, voire être identiques entre eux de manière à servir d'éléments de rechange. Des systèmes d'écran 14 peuvent également être commercialisés indépendamment de la monture 7 pour permettre aux utilisateurs du masque de remplacer leur écran.

Avantageusement, le corps 8 de la monture 7 est davantage déformable que le système d'écran 14. Le corps 8 de la monture 7 peut notamment être constitué d'un matériau plastique moins dur qu'un matériau plastique constituant le support 13. Le système d'écran 14 peut être considéré comme un ensemble indéformable comparativement au corps de la monture. Ainsi, seule la monture 7 est destinée à se déformer pour assembler ou désassembler le système d'écran à la monture. La souplesse du corps 8 de la monture 7 est donc mise à profit à la fois pour offrir un appui confortable de la monture 7 sur le visage et pour permettre l'assemblage et/ou le désassemblage du système d'écran 14. Par exemple, le corps 8 peut comprendre une résistance à la traction inférieure ou égale à 40MPa selon la norme ASTM D412. Le support 13 peut comprendre une résistance à la traction supérieure ou égale à 50MPa selon la norme ASTM D638.

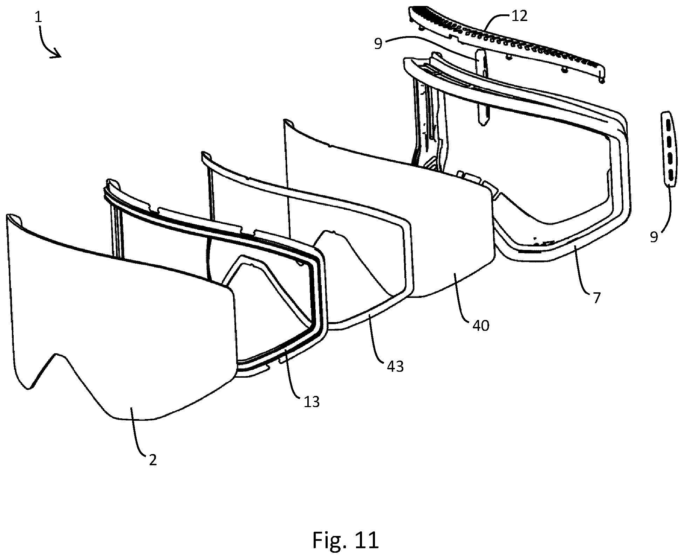

La figure 11 illustre un mode de réalisation dans lequel le masque 1 comprend un deuxième écran 40. Le deuxième écran 40 peut être fixé au support 13, à l'arrière de l'écran 2 précédemment décrit, dit « premier écran 2 ». Le deuxième écran 40 s'étend de préférence parallèlement au premier écran 2, à distance non nulle du premier écran 2. Une lame d'air est ainsi formée entre le premier écran et le deuxième écran. Cette lame d'air joue le rôle de barrière thermique et permet d'éviter la formation de buée dans le masque 1. Le premier écran 2 constitue donc un écran avant, et le deuxième écran 40 constitue un écran arrière.

Le support 13 comprend avantageusement une surface d'appui 41 (identifiée sur la figure 8) s'étendant sur un pourtour arrière du support 13, et contre laquelle le deuxième écran 40 est fixé, par exemple par collage. La surface d'appui 41 est formée sur une face arrière de l'embase 24. La surface d'appui 41 est en retrait vers l'avant par rapport à la surface arrière 26 précédemment décrite. Une face arrière du deuxième écran 40 peut ainsi s'étendre sensiblement dans le prolongement de la surface arrière 26. Le corps 8 de la monture peut éventuellement comprendre une languette 42 recouvrant un bord externe du deuxième écran. Le deuxième écran 40 peut être fixé au support 13 par de la colle ou au moyen d'un ruban adhésif 43 tel qu'un joint autocollant comme cela est représenté sur la figure 11. Ainsi, le support 13 sert non seulement à maintenir le premier écran 2 mais également le deuxième écran 40. Le deuxième écran 40 présente une surface strictement inférieure à la surface du premier écran 2. Le premier écran 2 s'étend davantage radialement que le deuxième écran 40.

Finalement, grâce à l'invention, on dispose d'un masque 1 de protection oculaire simple à fabriquer et à utiliser. Le moyen de fixation du système d'écran 14 à la monture 7 s'étend radialement et est ainsi particulièrement compact dans la direction axiale. Ceci permet de conserver un écran 2 proche du visage et un large champ de vision.

## Revendications

1. Masque (1) de protection oculaire comprenant une monture (7) et un système d'écran (14) fixé à la monture, le système d'écran comprenant un premier écran (2) et un support (13) fixé au premier écran, le premier écran recouvrant frontalement la monture et le support, **caractérisé en ce que** le support comprend :
- une surface avant (15) fixée contre une face arrière (16) du premier écran,
- une surface de retenue (23) s'étendant sensiblement parallèlement au premier écran à l'arrière du premier écran,
la surface de retenue et un bord externe (3) du premier écran formant ensemble une gorge radiale (22) coopérant avec la monture (7) pour retenir le système d'écran à la monture.

2. Masque (1) de protection oculaire selon la revendication précédente, **caractérisé en ce que** le support (13) comprend en outre une surface arrière (26) s'étendant en vis-à-vis de la monture (7), la surface de retenue (23) s'étendant entre la surface avant (15) du support et la surface arrière (26) du support.

3. Masque (1) de protection oculaire selon l'une des revendications précédentes, caractérisé que la monture (7) comprend une rainure radiale (27), la surface de retenue (23) étant insérée dans la rainure radiale.

4. Masque (1) de protection oculaire selon l'une des revendications précédentes, caractérisé que la surface de retenue (23) s'étend le long d'au moins 50%, de préférence au moins 80%, d'un bord externe (3) de l'écran.

5. Masque (1) de protection oculaire selon l'une des revendications précédentes, **caractérisé en ce que** la surface de retenue (23) comprend au moins une encoche (35), et **en ce que** la monture comprend au moins une protubérance (36) de forme complémentaire à la forme de l'au moins une encoche, l'au moins une protubérance étant emboîtée dans l'au moins une encoche.

6. Masque (1) de protection oculaire selon la revendication précédente, **caractérisé en ce que** l'au moins une encoche (35) comprend une entrée (37) rétrécie.

7. Masque (1) de protection oculaire selon la revendication 4 ou 5, **caractérisé en ce que** l'au moins une encoche (35) comprend :
- au moins une première encoche (35A) formée sur une portion de la surface de retenue destinée à s'étendre autour du nez,
- au moins une deuxième encoche (35B) formée sur une portion de la surface de retenue destinée à s'étendre en vis-à-vis d'une pommette, et
- au moins une troisième encoche (35C) formée sur une portion de la surface de retenue destinée à s'étendre en vis-à-vis du front.

8. Masque (1) de protection oculaire selon l'une des revendications précédentes, **caractérisé en ce que** la surface de retenue (23) s'étend au moins :
- dans une partie inférieure (31) du support destinée à s'étendre en vis-à-vis des pommettes et autour du nez,
- dans une partie supérieure (32) du support destinée à s'étendre en vis-à-vis du front,
- dans une partie latérale gauche (33) du support destinée à s'étendre en vis-à-vis d'un côté extérieur de l'œil gauche, et
- dans une partie latérale droite (34) du support destinée à s'étendre en vis-à-vis d'un côté extérieur de l'œil droit.

9. Masque (1) de protection oculaire selon l'une des revendications précédentes, **caractérisé en ce que** la monture (7) est davantage déformable que le système d'écran (14), seule la monture étant destinée à se déformer pour assembler ou désassembler le système d'écran à la monture.

10. Masque (1) de protection oculaire selon l'une des revendications précédentes, caractérisé en qu'il comprend une bande élastique configurée pour maintenir le masque de protection oculaire sur la tête d'une personne, la bande élastique étant fixée à la monture (7).

11. Masque (1) de protection oculaire selon l'une des revendication précédentes, **caractérisé en ce que** l'ensemble du bord externe (3) du premier écran (2) est visible depuis l'extérieur du masque.

12. Masque (1) de protection oculaire selon l'une des revendication précédentes, **caractérisé en ce que** le système d'écran comprend un deuxième écran s'étendant parallèlement au premier écran à l'arrière du premier écran, le deuxième écran étant maintenu par le support (13).
